(19) Europäisches Patentamt
European Patent Office
Office européen des brevets

(11) **EP 3 065 072 A1**

(12) **EUROPEAN PATENT APPLICATION**

(43) Date of publication:
**07.09.2016 Bulletin 2016/36**

(51) Int Cl.:
***G06F 19/00*** *(2011.01)*

(21) Application number: **16156616.1**

(22) Date of filing: **22.02.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **04.03.2015 KR 20150030384**

(71) Applicant: **Samsung Electronics Co., Ltd.
Gyeonggi-do 16677 (KR)**

(72) Inventors:
• **KIM, Ha Young
Gyeonggi-do (KR)**
• **WOO, Kyoung Gu
Seoul (KR)**

(74) Representative: **Arnold + Siedsma
Bezuidenhoutseweg 57
2594 AC The Hague (NL)**

(54) **APPARATUS AND METHOD FOR PROVIDING RELIABILITY FOR COMPUTER AIDED DIAGNOSIS**

(57) An apparatus for providing reliability for Computer Aided Diagnosis (CAD), including: a raw data collector configured to collect raw data containing an image acquired by a probe; an image reliability determiner configured to determine a reliability level of the image using the collected raw data; and a reliability provider configured to provide a user with the determined reliability of the image.

FIG. 1

**Description**

CROSS-REFERENCE TO RELATED APPLICATION(S)

This application claims benefit from Korean Patent Application No. 10-2015-0030384, filed on March 4, 2015, in the Korean Intellectual Property Office, the entire disclosure of which is incorporated herein in its entirety by reference.

BACKGROUND

1. Field

The following description relates to a Computer Aided Diagnosis (CAD) and, more particularly, to an apparatus and method for providing reliability for CAD.

2. Description of the Related Art

In the medical industry, Computer Aided Diagnosis (CAD) has been used to analyze medical images as a way of diagnosing a patient. Specifically, CAD techniques may be used to analyze various medical images to detect a lesion, classify whether a detected lesion is benign/malignant, and provide a doctor with the result. For example, in the case of ultrasonic diagnosis, a doctor acquires ultrasonic images in real time by moving a probe while in contact with a patient's body, and detects and determines a lesion or a suspicious area by checking ultrasonic images displayed on a screen with bare eyes. However, different medical images may be acquired by a probe according to environment, such as speed at which the doctor moves the probe on the patient's body and a degree to which the probe is in contact with the patient's body, thereby leading to different CAD results.

SUMMARY

According to an aspect of an exemplary embodiment, an apparatus for providing reliability information for Computer Aided Diagnosis (CAD), includes a raw data collector configured to collect raw data, the raw data including an image acquired by a probe; an image reliability determiner configured to determine a reliability level of the image using the collected raw data; and a reliability provider configured to provide a user with reliability information corresponding to the determined reliability level of the image.
The raw data collector may be further configured to collect the raw data from at least one from among a sensor installed in the probe or a diagnostic device, a wearable device worn by the user, and a device installed within a diagnostic screening room.
The image reliability determiner may be further configured to generate analysis information based on the collected raw data, and to determine the reliability level of the image based on the generated analysis information.
The analysis information may include at least one from among a moving speed of the probe, a degree of blurriness of the image, a measurement depth of the probe, and an angular velocity of the probe.
The image reliability determiner may be further configured to generate the analysis information based on a difference between the image and a previous image.
The image reliability determiner may be further configured to calculate a reliability level of the image by applying the generated analysis information to a calculation algorithm.
The image reliability determiner may be further configured to determine the reliability level of the image by searching a reliability database using the analysis information.
The reliability information may include at least one from among visual information or non-visual information, and the reliability provider may be further configured to output the reliability information to an output device.
The visual information may further include at least one from among color information corresponding to the determined reliability level of the image, graph information corresponding to the determined reliability level of the image, and numeric information having a value indicative of the determined reliability level of the image.
The non-visual information may further include at least one from among a predefined acoustic signal corresponding to the determined reliability level of the image, a predefined vibration signal corresponding to the determined reliability level of the image, and a voice signal regarding a numerical value indicative of the determined reliability of the image.
The output device may further include at least one from among the probe, a diagnostic device, a diagnostic display device, a directional speaker, a wearable device, and a haptic device.
According to another aspect of an exemplary embodiment, a method for providing reliability information for Computer Aided Diagnosis (CAD), includes collecting raw data, the raw data including an image acquired by a probe;

determining a reliability level of the image based on the collected raw data; and providing a user with reliability information corresponding to the determined reliability level of the image.

The determining of a reliability level of the image may include generating analysis information based on the collected raw data and determining the reliability level of the image based the generated analysis information.

The analysis information may include at least one from among a moving speed of the probe, a degree of blurriness of the image, measurement depth of the probe, and an angular velocity of the probe.

The reliability information may further include at least one from among visual information or non-visual information; and the providing of the reliability information may further include outputting the reliability information to an output device.

According to yet another aspect of an exemplary embodiment, an apparatus for providing diagnosis reliability information for Computer Aided Diagnosis (CAD), includes a raw data collector configured to collect raw data, the raw data including an image acquired by a probe; a diagnosis reliability determiner configured to determine a diagnosis reliability level corresponding to a diagnosis performed by a diagnostic device using the collected raw data; and a reliability provider configured to provide a user with diagnosis reliability information corresponding to the determined diagnosis reliability level.

The apparatus may further include: an image reliability determiner configured to generate analysis information based on the collected raw data, and determine an image reliability level of the image based on the generated analysis information.

The apparatus may further include a diagnostic algorithm selector configured to select a diagnostic algorithm to be applied to the image.

The diagnosis reliability level of the diagnosis may include at least one of a reliability level of a diagnostic algorithm applied to the image and a reliability level of a diagnostic result.

The diagnosis reliability information may further include at least one from among visual information or non-visual information, and the reliability provider may be further configured to output the diagnosis reliability information to an output device.

According to a further aspect of an exemplary embodiment, a method for providing diagnosis reliability information for Computer Aided Diagnosis (CAD) includes collecting raw data, the raw data including an image acquired by a probe; determining a diagnosis reliability level of a diagnosis performed by a diagnostic device based on the collected raw data; and providing a user with diagnosis reliability information corresponding to the determined diagnosis reliability level.

The method may further include generating analysis information based on the collected raw data; and determining an image reliability level of the image based on the generated analysis information.

The method may further include selecting a diagnostic algorithm to be applied to the image based on the determined reliability level of the image.

According to a further aspect of an exemplary embodiment, a method of providing diagnostic reliability information for Computer Aided Diagnosis (CAD), includes collecting raw data, the raw data including an image acquired by a probe; determining an image reliability level based on the collected raw data; determining a diagnosis reliability level based on at least one from among the image reliability level and the collected raw data; providing a user with at least one from among diagnosis reliability information corresponding to the diagnostic reliability level, and image reliability information corresponding to the image reliability level.

Determining the diagnosis reliability level may further include: selecting a diagnostic algorithm based on the image reliability level; and determining the diagnosis reliability level by applying the selected diagnostic algorithm to at least one from among the image reliability level and the raw data.

Other features and aspects may be apparent from the following detailed description, the drawings, and the claims.

BRIEF DESCRIPTION OF THE DRAWINGS

FIG. 1 is a block diagram illustrating an apparatus for providing reliability according to an exemplary embodiment.
FIGS. 2A and 2B are block diagrams illustrating an image reliability determiner shown in FIG. 1 according to exemplary embodiments.
FIGS. 3A to 3D illustrate examples in which a reliability provider displays reliability according to exemplary embodiments.
FIG. 4 is a block diagram illustrating an apparatus for providing reliability according to another exemplary embodiment.
FIGS. 5A and 5B are block diagrams illustrating a diagnosis reliability determiner according to exemplary embodiments.

FIG. 6 is a block diagram illustrating an apparatus for providing reliability according to yet another exemplary embodiment.

FIG. 7 is a block diagram illustrating an apparatus for providing reliability according to yet another exemplary embodiment.

FIG. 8 is a flowchart illustrating a method for providing reliability according to an exemplary embodiment.

FIG. 9 is a flowchart illustrating a method for providing reliability according to another exemplary embodiment.

FIG. 10 is a flowchart illustrating a method for providing reliability according to yet another exemplary embodiment.

FIG. 11 is a flowchart illustrating a method for providing reliability according to yet another exemplary embodiment.

FIG. 12 is a flowchart illustrating a method for providing reliability according to yet another exemplary embodiment.

Throughout the drawings and the detailed description, unless otherwise described, the same drawing reference numerals will be understood to refer to the same elements, features, and structures. The relative size and depiction of these elements may be exaggerated for clarity, illustration, and convenience.

## DETAILED DESCRIPTION

The following description is provided to assist the reader in gaining a comprehensive understanding of the methods, apparatuses, and systems described herein. Accordingly, various changes, modifications, and equivalents of the methods, apparatuses, and/or systems described herein will be suggested to those of ordinary skill in the art. Also, descriptions of well-known functions and constructions may be omitted for increased clarity and conciseness.

Hereinafter, exemplary embodiments of an apparatus and method for providing reliability for Computer Aided Diagnosis (CAD) will be described with reference to drawings.

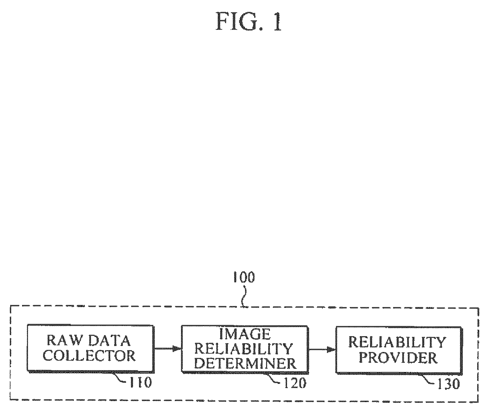

FIG. 1 is a block diagram illustrating an apparatus for providing reliability according to an exemplary embodiment.

According to an exemplary embodiment, an apparatus 100 for providing reliability may be included in a diagnostic device which performs CAD using medical images, or may be included in an additional external device so as to be connected to the diagnostic device over wired/wireless communication.

The medical images may be an ultrasonic images acquired by a probe, and this assumption applies to the following descriptions. However, aspects of the present disclosure are not limited thereto, and in some exemplary embodiments, medical images may include images acquired by a Computed Tomography (CT) device, a Magnetic Resonance Imaging (MRI) device, an X-ray device, a Positron Emission Tomography (PET) device, a Single Photon Emission Computed Tomography (SPECT) device, and the like.

Referring to FIG. 1, according to an exemplary embodiment, the apparatus 100 includes a raw data collector 110, an image reliability determiner 120, and a reliability provider 130.

The raw data collector 110 collects data required for analysis of a reliability level of an image acquired by a probe.

The raw data may include an ultrasonic image acquired by the probe. In addition, the data may include various types of sensor data which are measurements obtained by various sensors, including a speed sensor, a location sensor, a speech sensor, a tactile sensor, a tensor sensor, a temperature sensor, and a camera module, which are installed in the probe, a diagnostic device, a diagnostic display device, a wearable device of a user, and a diagnostic screening room.

Based on the collected raw data, the image reliability determiner 120 may determine a reliability level of an image acquired by a probe.

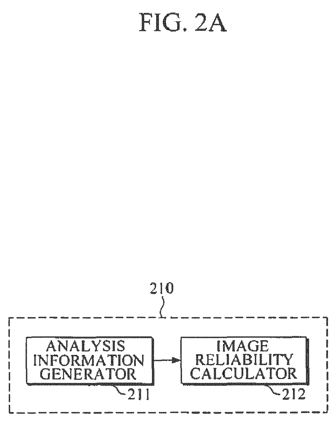

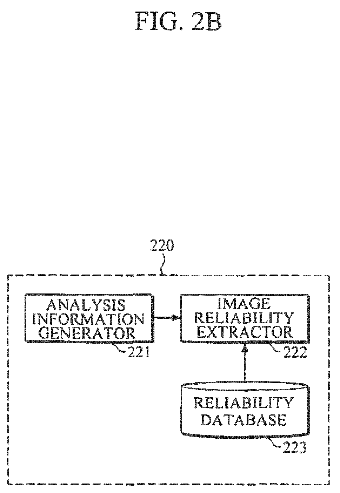

FIGS. 2A and 2B are block diagrams illustrating an image reliability determiner 120 according to exemplary embodiments. With reference to FIGS. 2A and 2B, examples 210 and 220 of the image reliability determiner 120 are described in detail.

Referring to FIG. 2A, an image reliability determiner 210 according to an exemplary embodiment includes an analysis information generator 221 and a reliability calculator 212.

The analysis information generator 221 generates analysis information required for analysis of an image reliability level based on the collected raw data. The analysis information may be parameter information to be input to a predefined calculation algorithm, and may include one or more of the following: a probe's moving speed, a degree of artifact in an image, a degree of blurriness in an image, a measurement depth of the probe, and an angular velocity of the probe. However, aspects of the present disclosure are not limited thereto, and types of analysis information to be generated by the calculation algorithm may be adjusted.

For example, the analysis information generator 211 may generate analysis information, such as a probe's moving speed, based on a difference between a current image and a previous image in images continuously acquired by the probe. The difference between the current image and the previous image may include a difference in image intensity per pixel between the current image and the previous image, a difference in histogram between the current image and the previous image, a similarity level in histogram between the current image and the previous image, and a difference in primary information between the current image and the previous image. For example, if a pixel value indicating darkness of an image is greater than a specific threshold, it may be possible to determine that the probe is off from a contact surface and to acquire information on a measurement depth of the probe based on the determination.

In another example, in the case where an image of a user manipulating a probe is acquired by a camera module installed in a diagnostic device or a diagnostic screening room, the analysis information generator 211 may acquire the probe's moving speed by analyzing the image.

In yet another example, the analysis information generator 211 may utilize various schemes, such as computer vision and machine learning, to generate analysis information.

In the case where necessary analysis information exists in raw data received from various sensors, the analysis information generator 211 may omit the operation of calculating analysis information, but use the collected raw data as analysis information.

For example, in the case where information on a moving speed of a probe is collected as raw data from a speed sensor installed in the probe, the analysis information generator 211 may not calculate the probe's moving speed based on an image acquired by the probe or an image collected from an external camera module. In addition, a depth mode which is set by default in the probe may be collected may be selected as raw data, and, in this case, the analysis information generator 211 may omit an operation of calculating information on measurement depth information of the probe.

The image reliability calculator 212 may calculate an image reliability level by applying analysis information to a calculation algorithm. The calculation algorithm may be one of various algorithms including but not limited to computer vision, machine learning, a linear function, a non-linear function, and a regression.

For example, the following Equation 1 is an example of an algorithm used for calculating an image reliability level using various types of analysis information.

【Equation 1】

$$Y = af(speed) + bf(blurrniess) + cf(depth) + \cdots$$

In Equation 1, Y denotes an image reliability level, f denotes a linear or non-linear function for analysis information, i.e., speed, blurriness, and depth. In addition, a, b, and c are predefined constants indicating weights corresponding to speed, blurriness, and depth, respectively. If necessary, a weight for each analysis information item may be adjusted. Types of analysis information used in calculation of an image reliability level may be determined beforehand. For example, an image reliability level may be determined only using a moving speed of a probe, as shown in the following Equation 2.

【Equation 2】

$$Y = af(speed) + c$$

An image reliability level may be represented in percentage points (e.g., 85%) converted from a value calculated by a calculation algorithm. Alternatively, among a plurality of predefined levels (e.g., levels 1 to 10), a value calculated by a calculation algorithm may be represented as any one level (e.g., level 8). However, aspects of exemplary embodiments are not limited thereto, and an image reliability level may be represented in various ways.

Referring to FIG. 2B, the image reliability determiner 220 according to another exemplary embodiment includes an analysis information generator 221, a reliability extractor 222, and a reliability database 223.

The analysis information generator 221 performs the same function as those of the analysis information generator 211 of FIG. 2A, and thus, detailed descriptions thereof are omitted.

When analysis information is generated, the reliability extractor 222 may extract an image reliability level corresponding to the analysis information from the reliability database 223.

As shown in Table 1 or Table 2, the reliability database 223 may store information where image reliability levels are mapped to analysis information in a table form. The reliability database 223 may be generated beforehand through a preprocessing procedure that is implemented by applying various types of analysis information to various kinds of calculation algorithms, analyzing the calculation results, and then determining an optimal image reliability level.

For example, Table 1 shows image reliability levels which are mapped beforehand to analysis information, that is, speed, blurriness, and depth. Referring to Table 1, if analysis information generated by the analysis information generator 221 shows that a probe's moving speed is 35 ms, that a degree of blurriness is 7%, and that depth is 85%, the reliability extractor 222 may extract an image reliability level of 90% from the reliability database 223.

[Table 1]

| Probe speed (v) | Blurriness | Depth | Image reliability |
|---|---|---|---|
| 0≤v<10 ms | 1% | Less than 75% | 60% |
| 10 ms≤v≤20 ms | < 2% | Less than 80% | 70% |
| 20 ms≤v≤30 ms | <5% | Less than 90% | 80% |
| 30 ms≤v≤40 ms | < 10% | Less than 95% | 90% |
| 40 ms≤v≤50 ms | < 15% | Less than 100 | 100% |

Table 2 shows an example of image reliability levels which are mapped beforehand to a probe's speed. For example, if analysis information generated by the analysis information generator 221 shows that a probe's speed is 27ms, the reliability extractor 222 may extract an image reliability level of 80%.

[Table 2]

| Probe speed (v) | Image reliability |
|---|---|
| 0≤v≤10 ms | 60% |
| 10 ms≤v≤20 ms | 70% |
| 20 ms≤v≤30 ms | 80% |
| 30 ms≤v≤40 ms | 90% |
| 40 ms≤v≤50 ms | 100% |

Again referring to FIG. 1, the reliability provider 130 provides a user with an image reliability level determined by the image reliability determiner 120. The reliability provider 130 generates visual information or non-visual information of the determined image reliability level, and provides the user with the generated information by outputting the generated information to an output device.

The visual information may include at least one of color or graph information corresponding to the image reliability level and numeric or character information corresponding to the image reliability level.

As shown in Table 3, image reliability levels may be divided into multiple sections and each section may be defined by a different color. If the image reliability is divided minutely, color information may be defined in a manner of changing from dark red to dark green seamlessly.

[Table 3]

| Image reliability | Color information |
|---|---|
| Less than 50% | Red |
| greater than 50%, less than 80% | Orange |
| greater than 80% and up to 100% | Green |

In the example case where the image reliability determiner 120 determines image reliability levels of 79%, 40%, and 85% respectively for images (e.g., images 1, 2, and 3) continuously received from a probe, the reliability provider 130 may, for example, output color information in a sequence of orange, red, and green to an output device.

In addition, the non-visual information may include at least one of an acoustic or vibration signal corresponding to an image reliability level and a voice signal corresponding to the image reliability level.

For example, intensity of an acoustic signal (i.e., beep sound) or a vibration signal and the number of occurrence thereof may be set beforehand according to image reliability sections, as shown for example in Table 4. As described above, image reliability levels may be divided into various sections. The narrower the interval between sections is, the more or the less the intensity of an acoustic signal or a vibration signal may increase or decrease continuously. In some exemplary embodiments, the intensity of a signal or the number of occurrence of the signal may be defined to be disproportional to an image reliability level. However, aspects of the present disclosure are not limited thereto.

[Table 4]

| Image reliability | Intensity of acoustic/vibration signal | Number of occurrence of acoustic/vibration signals |
|---|---|---|
| Less than 50% | High | 3 |
| Greater than 50%, less than 80% | Medium | 2 |
| Greater than 80% and up to 100% | Low | 1 |

The output device may include a probe, a diagnostic device, a diagnostic display device, a directional speaker, a wearable device that a user puts on, and a device installed in a specif location within a diagnostic screening room.

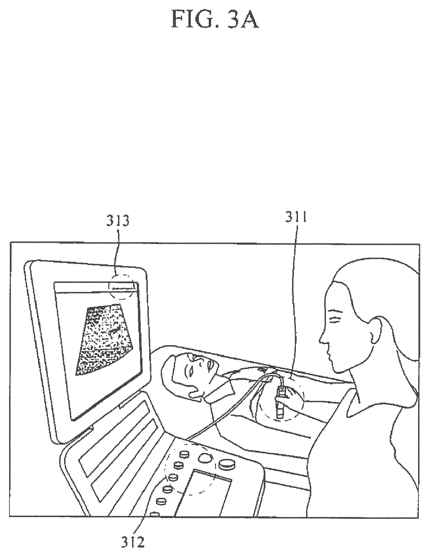

FIGS. 3A to 3D are examples in which a reliability provider shown in FIG. 1 displays an image reliability level.

With reference to FIG. 1 and FIGS. 3A to 3D, there are provided examples in which the reliability provider 130 outputs a determined image reliability level to an output device.

FIG. 3A illustrates a process in which a user examines a patient in a diagnostic screening room. The user acquires images continuously from a probe 311 by moving a probe 311 while in contact with a body of the patient. In this case, a diagnostic device 312 performs diagnosis by applying a diagnostic algorithm to an acquired image, and then outputs the acquired image and a diagnostic result thereof to a diagnosis display device 313.

As such, when a user carries out an examination process, the raw data collector 110 of the apparatus 100 collects various types of information generated during examination of a patient as analysis information. For example, the information generated during examination may include an image acquired by the probe 311 and sensor data received from sensors which are embedded or attached in the probe 311, the diagnostic device 312, the diagnostic display device 313, and the like.

The image reliability determiner 120 determines a reliability level of an image acquired by the probe 311 based on the collected raw data.

The reliability provider 130 outputs the determined image reliability level to an output device. Referring to FIG. 3A, the output device may be the probe 311, the diagnostic device 312, and the diagnostic display apparatus 313.

In some exemplary embodiments, when an image reliability level is displayed on the output device during a diagnostic process, a user may adjust speed of a probe, a degree to which the probe is in contact with an examination part of a patient's body, a measurement depth mode, and a location of an area where a probe is in contact with the examination part of the patient body. As a result, a more accurate image may be acquired.

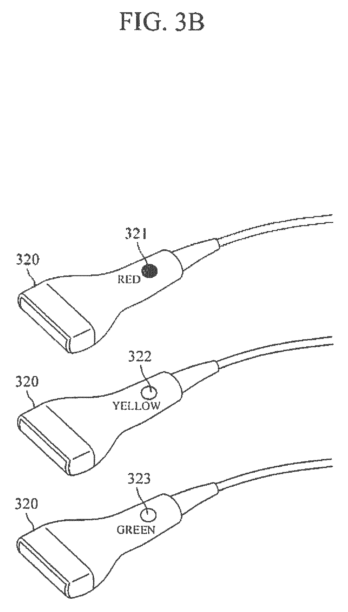

FIG. 3B illustrates a case where an image reliability level is output in a probe. As illustrated in FIG. 3B, different colors 321, 322, or 323 may be output at a specific portion of the probe 320 according to a determined image reliability level. However, it is merely exemplary, and the colors 321, 322, and 323 may be output on a specific portion of a diagnostic device or a specific location of a diagnostic screening room.

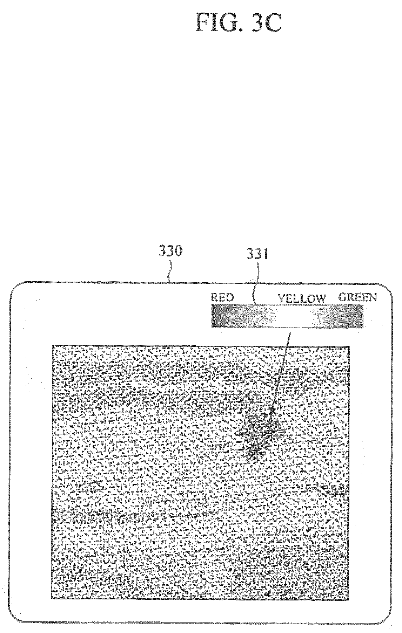

FIG. 3C illustrates an example in which an image reliability level is displayed in a graph form in a diagnostic display device.

Referring to FIG. 3C, red, orange, yellow, and green are all displayed in visual information in a graph form 331, but color to be displayed may change according to a continuously received image. For example, in the case where a color corresponding to a reliability level of a previous image is red and a color corresponding to a reliability level of the current image is green, red may become blurred and green becomes rich when the previous image is switched to the current image. However, it is merely exemplary, and a reliability level of an image may be displayed in various ways.

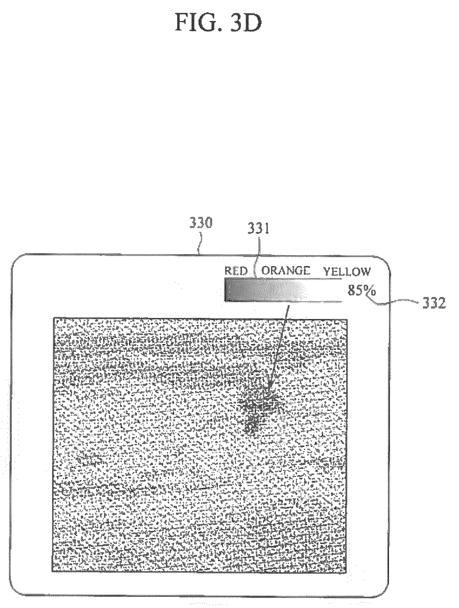

FIG. 3D illustrates a case in which graph information 331 and numeric information 332 of an image reliability level are displayed together in a diagnostic display device 330.

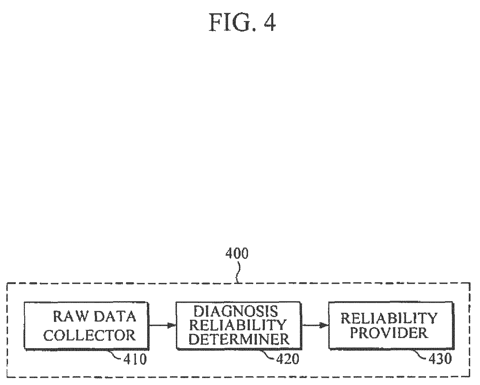

FIG. 4 is a block diagram illustrating an apparatus for providing reliability according to another exemplary embodiment.

Referring to FIG. 4, an apparatus 400 for providing reliability includes a raw data collector 410, a diagnosis reliability determiner 420, and a reliability provider 430.

The raw data collector 410 collects various types of raw data, as described above.

Based on the collected raw data, the diagnosis reliability determiner 420 may determine a reliability level of diagnosis performed by a diagnostic device (hereinafter referred to as a diagnosis reliability level). The diagnosis reliability level may include a reliability level of a diagnostic algorithm or a reliability level of a diagnostic result obtained by using a diagnostic algorithm. The reliability level of a diagnostic algorithm may indicate whether the diagnostic algorithm currently applied to the diagnostic device is suitable for the current image. The diagnostic algorithm may be one of AdaBoost, Deformable Part Models (DPM), Support Vector Machine (SVM), Decision Tree, Deep Belief Network (DBN), and Convolutional Neural Network (CNN).

The reliability provider 430 provides a diagnosis reliability level determined by the diagnosis reliability determiner 420. The reliability provider 430 may generate visual information or non-visual information of the determined diagnosis reliability level, and output the generated information to an output device.

The visual information may include at least one of color or graph information corresponding to an image reliability level and numeric or character information corresponding to the image reliability level. With respect to the color information, a plurality of sections of image reliability levels may be divided into a plurality of sections, and each section may be defined by a different color, as illustrated in the above Table 3. The interval between sections may be set differently as desired.

As illustrated in FIGS. 3A to 3D, the reliability provider 430 may output generated visual information or non-visual information to an output device, such as a probe, a diagnostic device, and a diagnostic display device. Detailed descriptions thereof are omitted.

FIGS, 5A to 5B are detailed block diagrams illustrating a diagnosis reliability determiner 420 according to exemplary embodiments.

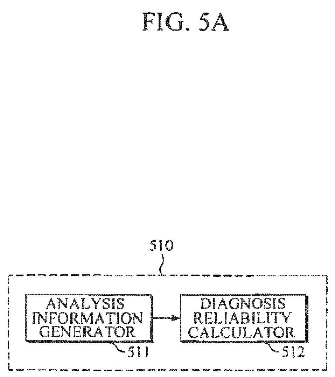

Referring to FIG. 5A, a diagnosis reliability determiner 510 according to an exemplary embodiment includes an analysis information generator 511 and a diagnosis reliability calculator 512.

The analysis information generator 511 may generate analysis information by using raw data collected by the raw data collector 410. The analysis information may include a probe's speed, a degree of blurriness, depth information, and information on size of a region of interest (ROI).

As described above, the analysis information generator 511 may generate analysis information based on a difference between the current image and a previous image. Alternatively, in the case where raw data corresponding to analysis information is collected by various sensors installed in a probe, a diagnostic device, a diagnostic display device, a wearable device, and the like, the analysis information may include a probe's moving speed, a degree of blurriness, depth information, and information on size of an ROI. The operation of generating analysis information may be omitted. For example, information on size of an ROI may be information on size of an ROI that is detected by a diagnostic device from the current image.

The diagnosis reliability calculator 512 may calculate a diagnosis reliability level by applying a predefined calculation algorithm to the generated analysis information. In some exemplary embodiments, the predefined calculation algorithm may be defined in the same or similar way as Equation 1 or Equation 2.

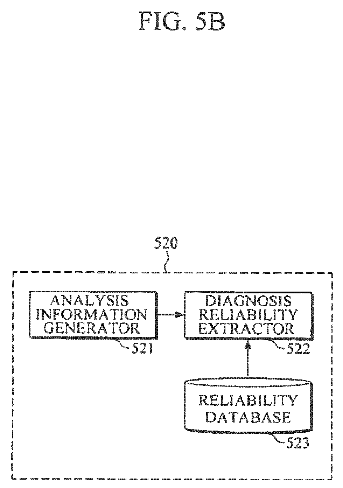

Referring to FIG. 5B, a diagnosis reliability determiner 420 according to another exemplary embodiment includes an analysis information generator 521, a diagnosis reliability extractor 522, and a reliability database 523.

The analysis information generator 521 performs the same functions as those of the analysis information generator 511 included in FIG. 5A, and thus, detailed descriptions thereof are omitted.

Based on the generated analysis information, the diagnosis reliability extractor 522 may extract a diagnosis reliability level corresponding to the generated analysis information from the reliability database 523. The diagnosis reliability level may include a reliability level of a diagnostic algorithm or a reliability level of a diagnostic result.

Table 5 is an example in which reliability level of a diagnostic algorithm (i.e., CNN) according to speed of a probe is stored beforehand in the reliability database 523. Referring to Table 5, the more slowly a probe moves, the higher the reliability level of CNN becomes. CNN is an algorithm that has a slow analysis speed but relatively high accuracy in analysis. However, it is merely exemplary, and reliability levels of diagnostic algorithms may be defined differently according to analysis information due to characteristics of the diagnostic algorithms.

[Table 5]

| Probe speed (v) | Diagnostic algorithm (CNN) |
|---|---|
| $0 \leq v < 10$ ms | 100% |
| 10 ms $\leq v \leq 20$ ms | 90% |
| 20 ms $\leq v \leq 30$ ms | 80% |
| 30 ms $\leq v \leq 40$ ms | 70% |
| 40 ms $\leq v \leq 50$ ms | 60% |

Similarly, diagnostic result reliability levels may be stored beforehand in the reliability database 523 as information which is in a table form where diagnostic result reliability levels are mapped to analysis information. The diagnosis reliability extractor 522 may extract a reliability level of a diagnostic result by reference to the mapping information stored in the reliability database 523.

The reliability extractor 522 may extract a reliability level of a diagnostic algorithm, and then extract a reliability level of a diagnostic result based on the extracted reliability level of the diagnostic algorithm. The reliability database 523 may have already stored information where diagnostic algorithm reliability levels and diagnostic result reliability levels are mapped.

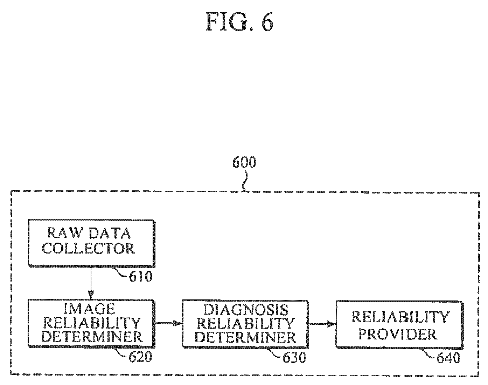

FIG. 6 is a block diagram illustrating an apparatus for providing reliability according to another exemplary embodiment.

Referring to FIG. 6, an apparatus 600 includes a raw data collector 610, an image reliability determiner 620, a diagnosis reliability determiner 630, and a reliability provider 640.

The raw data collector 610 performs the same functions of the raw data collector 110 included in the apparatus 100 and of the raw data collector 410 included in the apparatus 400. Thus, and thus detailed descriptions of the raw data collector 610 are omitted.

When raw data is collected by the raw data collector 610, the image reliability determiner may generate analysis information based on the collected raw data. A type of analysis information to be generated may be predetermined as desired. If there is raw data corresponding to analysis information, an operation of generating analysis information may be omitted.

When the analysis information is generated, the image reliability determiner 620 may determine a reliability level of an image acquired by a probe based on the generated analysis information.

In one embodiment, the image reliability determiner 620 may calculate an image reliability level by applying the generated analysis information to a predefined calculation algorithm. In another embodiment, information in a table form where image reliability levels are mapped to analysis information may be stored in a reliability database through a preprocessing procedure. When analysis information is generated, the image reliability determiner 620 may extract, from the reliability database, an image reliability level mapped to the generated analysis information.

When an image reliability level is determined by the image reliability determiner 620, the diagnosis reliability determiner 630 may determine a diagnosis reliability level based on the determined image reliability level. Information where diagnosis reliability levels are mapped to image reliability levels may be stored beforehand in the reliability database, and the diagnosis reliability determiner 630 may determine a diagnosis reliability level by reference to the reliability database.

Although in some exemplary embodiments a diagnosis reliability level according to an image reliability level, for example, a reliability level of a diagnostic algorithm, is equal to a determined image reliability level, the diagnosis reliability level may be defined differently according to a type and characteristics of the currently applied diagnostic algorithm.

The reliability provider 640 may output the determined image reliability level or the diagnosis reliability level to an output device. As described above, the reliability provider 640 may generate visual information or non-visual information of the image reliability level or the diagnosis reliability level, and output the generated information to an output device.

In some exemplary embodiments, reliability provider 640 may output the image reliability level and the diagnosis reliability level at the same time to a single output device. For example, when a current image is displayed on a diagnostic display device, the reliability provider 640 may output an image reliability level at a specific position on the diagnostic display device. In addition, if for example a diagnostic device detects an ROI from the current image, generates a diagnostic result about whether the ROI is classified as benign/malignant, and outputs the diagnostic result on the diagnostic display device, the reliability provider 640 may output a diagnostic reliability level at the same time along with the image reliability level.

In other exemplary embodiments, the reliability provider 640 may output an image reliability level and a diagnosis reliability level to different output devices. For example, when an image is acquired by a probe and output to a diagnostic display device, the reliability provider 640 may display an image reliability level in a probe, as shown in the example of FIG. 3B. Then, when a diagnostic result of the image is output to the diagnostic display device, the reliability provider 640 may display a diagnosis reliability level in the diagnostic display device.

However, an example of outputting an image reliability level and diagnosis reliability level is not limited thereto. For example, an image reliability level may be output in a form of color information, and diagnosis reliability may be output in a form of graph. In another example, an image reliability level may be output in a form of non-visual information, and a diagnosis reliability level may be output in a form of visual information. As such, a reliability level of each item may be output by combining two or more types of visual or non-visual information or may be output to two or more output devices.

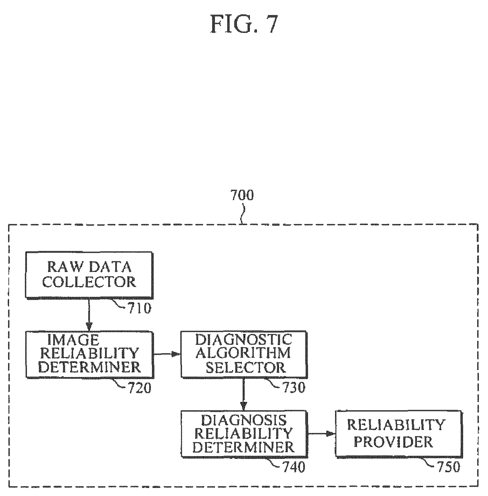

FIG. 7 is a block diagram illustrating an apparatus for providing reliability according to another exemplary embodiment.

Referring to FIG. 7, an apparatus 700 for providing reliability includes a raw data collector 710, an image reliability determiner 720, a diagnostic algorithm selector 730, a diagnostic reliability determiner 740, and a reliability provider 750. That is, the apparatus 700 includes the diagnostic algorithm selector 730 in addition to the elements of the apparatus 600 shown in FIG. 6. Each element included apparatus 700 perform the same functions as a corresponding element included in the apparatus 600, and thus, detailed descriptions thereof are omitted.

The raw data collector 710 may collect various kinds of data generated during diagnosis. For example, the raw data may include an image acquired by a probe, sensor data received from sensors installed in various devices, and information on an image captured by a camera module that captures the inside of a diagnostic screening room.

Based on the raw data, the image reliability determiner 720 may generate analysis information necessary for analysis of reliability. The analysis information may be a parameter value of a calculation algorithm for calculating an image reliability level. In other exemplary embodiments, the analysis information may be analysis information previously stored in a reliability database or may be attribute values that are required to refer to a table where image reliability levels are mapped to analysis information.

In the case where there is a reliability database, when the analysis information is generated, the image reliability determiner 720 may extract an image reliability level corresponding to the generated analysis information from the reliability database. In this case, the image reliability level may be determined very quickly. In other exemplary embodiments, in the case where there is no reliability database, the image reliability determiner 720 may calculate an image reliability level by applying the generated analysis information to a calculation algorithm.

When a reliability level of an image currently received from a probe is determined, the diagnostic algorithm selector 730 may select a diagnostic algorithm, which is suitable for performing diagnosis in a diagnostic device, according to the determined reliability level.

In the CAD, there are various diagnostic algorithms that analyzes an image and performs diagnosis, such as detection, tracking, and determination. The diagnostic algorithms may include, for example, AdaBoost, Deformable Part Models (DPM), Support Vector Machine (SVM), Decision Tree, Deep Belief Network (DBN), Convolutional Neural Network (CNN), and the like. Each diagnostic algorithm has various characteristics. For example, CNN algorithm may show relatively slow speed in diagnosis, but may generate a relatively accurate diagnostic result, so that it may be more useful when there is only a small difference between images captured by a slowly moving probe.

When images are continuously received from a probe, an image reliability level may be changed each time, and the diagnostic algorithm selector 730 may select a suitable diagnostic algorithm based on an image reliability level determined each time so as to generate a more accurate diagnostic result. In this case, information where image reliability levels are mapped to diagnostic algorithms respectively suitable therefor may be predefined. In this case, based on the mapping information, the diagnostic algorithm selector 730 may determine a diagnostic algorithm corresponding to an image reliability level to be a diagnostic algorithm that would be used in a diagnostic algorithm.

[Table 6]

| Image reliability | Diagnostic algorithm |
| --- | --- |
| less than 50% | CNN |
| more than 50%, less than 80% | SVM |
| more than 80%, up to 100% | DPM |

For example, referring to Table 6, in the case where a reliability level of the current image acquired by a probe is determined to be 49%, the diagnostic algorithm selector 730 selects CNN. Then, the diagnostic device may generate a diagnostic result by applying CNN to the current image. CNN is continuously used until the image reliability level is changed to belong to a different section. Then, if a reliability level of an acquired image goes up to 75% as a user changes speed of the probe or presses the probe against the examination part of a patient's body, the diagnostic algorithm selector 730 may change a diagnostic algorithm from CNN to SVM, and then the diagnostic device may perform diagnosis by applying SVM to the acquired image.

The diagnosis reliability determiner 740 may determine a reliability level of each diagnostic result obtained by using a diagnostic algorithm that is selected by the diagnostic algorithm selector 730. The diagnosis reliability determiner 740 may determine a reliability level of each diagnostic result by considering not only the aforementioned analysis information, but also an image reliability level and a reliability level of the selected diagnostic algorithm.

For example, a reliability level of a diagnostic result may be calculated by inputting parameter values indicative of analysis information, an image reliability level, a reliability level of a diagnostic algorithm, respectively, to a calculation algorithm like the aforementioned Equation 1. However, aspects of the present disclosure are not limited thereto, and, if there is a predefined mapping table in a reliability database, a reliability level of a diagnostic result may be extracted by reference to the mapping table.

The reliability provider 750 may generate visual information or non-visual information of the image reliability level, the diagnostic algorithm, and the diagnosis reliability level, and output the generated information to various output devices.

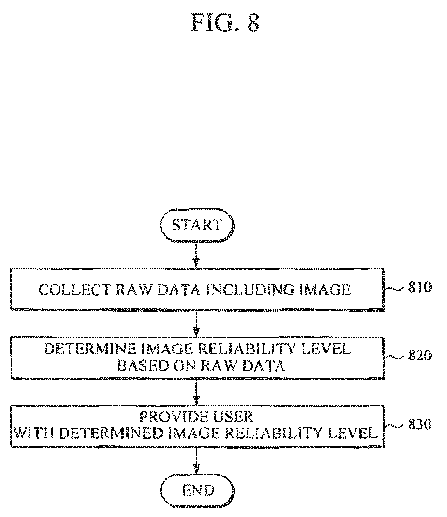

FIG. 8 is a flowchart illustrating a method for providing reliability according to an exemplary embodiment. FIG. 8 may be an example of a method implemented by the apparatus shown in FIG. 1 for providing reliability.

Referring to FIG. 8, the apparatus 100 collects raw data in 810. The raw data may include an image acquired by a probe, and various types of sensor data generated during diagnosis, such as speed of the probe, photographic depth mode information, an angular velocity, and the like.

Then, an image reliability level may be determined using the collected raw data in 820. The apparatus 100 may calculate an image reliability level using a predefined calculation algorithm. Alternatively, the apparatus 100 may extract an image reliability level by reference to a reliability database.

Then, the determined image reliability level is provided to a user in 830. The apparatus 100 generates visual information or non-visual information of the determined image reliability, and outputs the generated information to an output device. For example, in the case where an output device is a probe, a color indicative of a section corresponding to the image reliability level determined in 820 among colors predefined for different reliability sections may be output at a specific position on the probe. In other exemplary embodiments, on a screen of the diagnostic device where an image received from the probe is displayed, the apparatus 100 may display the determined image reliability level in a form a graph or may display a value indicative of the determined image reliability level.

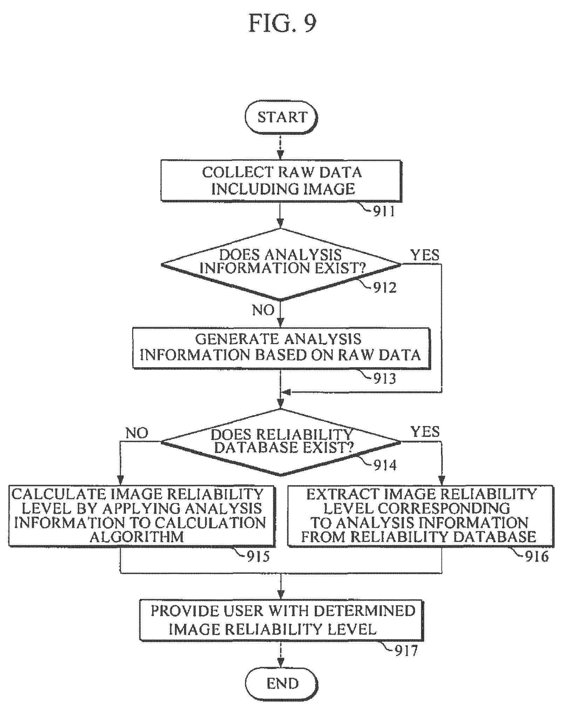

FIG. 9 is a flowchart illustrating a method for providing reliability according to another exemplary embodiment.

Referring to FIG. 9, the apparatus 100 collects raw data including an image acquired by a probe in 911.

Then, in 912, the apparatus 100 determines whether the collected raw data includes all analysis information necessary for analysis of an image reliability level. If some or all the necessary analysis information does not exist in the collected raw data, the apparatus 100 generates the non-existing analysis information based on the raw data in 913. Alternatively, if all the necessary analysis information exists, the apparatus 100 proceeds with the next operation in 914 without performing operation 913.

For example, suppose speed of a probe, a degree of blurriness, depth, and an angular velocity were analysis information necessary for analysis of an image reliability level. In this case, if a speed sensor is installed in the probe, speed of the probe may be collected as raw data. That is, the apparatus 100 does not generate probe speed information, but a degree of blurriness, depth, and an angular velocity based on raw data, for example, difference between images that are continuously received from the probe. Similarly, if a location sensor or a depth sensor is installed in the probe, the apparatus 100 may collect depth information and angular velocity information as raw data.

In 914, when the analysis information is generated, the apparatus 100 determines whether there is a reliability database that stores information where image reliability levels are mapped to the generated analysis information.

In 914, if there is a reliability database, the apparatus 100 extracts an image reliability level corresponding to the generated analysis information from the reliability database. In 915, if there is no reliability database, the apparatus 100 calculates an image reliability level by inputting the generated analysis information to a predefined calculation algorithm as a parameter.

In 917, the apparatus 100 provides a user with the image reliability level determined in 914 or 915 by outputting the image reliability level to various output devices.

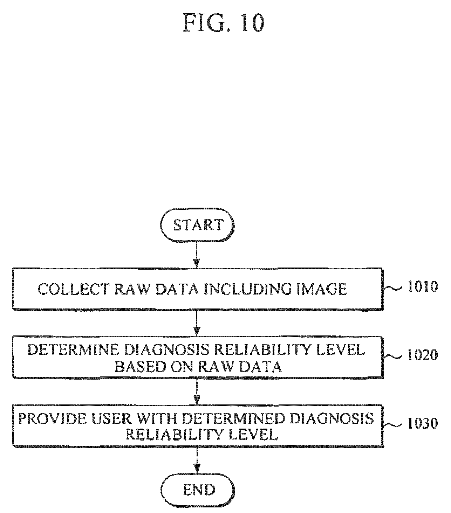

FIG. 10 is a flowchart illustrating a method for providing reliability according to another exemplary embodiment. FIG. 10 may be an example of a method implemented by the apparatus 400 shown in FIG. 4 for providing reliability.

Referring to FIG. 10, the apparatus 400 collects various types of raw data in 1010.

Based on the collected raw data, the apparatus 100 determines a reliability level of diagnosis performed by a diagnostic device in 1020. The diagnosis reliability level may include at least one of a reliability level of a diagnostic algorithm and a reliability level of a diagnostic result obtained by using the diagnostic algorithm. The reliability level of a diagnostic algorithm may indicate whether a diagnostic algorithm performing diagnosis in the diagnostic device is suitable for the current image acquired by a probe.

The reliability provider 400 may determine a diagnosis reliability level by applying analysis information to a reliability calculation algorithm or by referring to information stored in a reliability database.

Then, the apparatus 100 provides a user with the determined diagnosis reliability level in 1030. The determined diagnosis reliability level may be output to an output device in a visual or non-visual way.

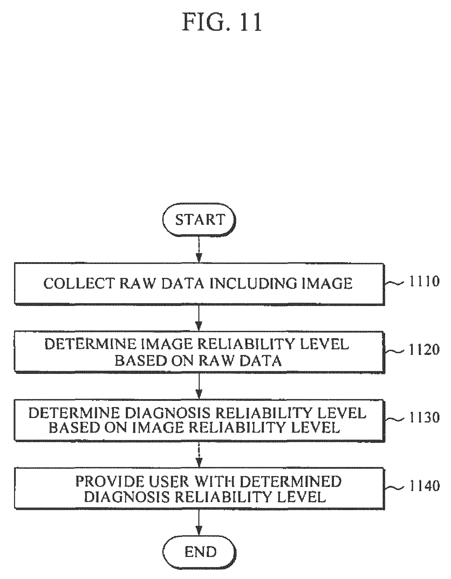

FIG. 11 is a flowchart illustrating a method for providing reliability according to another exemplary embodiment. FIG. 11 is an example of a method implemented by the apparatus 600 shown in FIG. 6 for providing reliability.

Referring to FIG. 11, the apparatus 600 collects raw data in 1110.

Based on the collected raw data, the apparatus 600 determines an image reliability level in 1120. Specifically, the apparatus 600 may generate analysis information based on the raw data, and determine an image reliability level using the analysis information.

In 1130, when a reliability level of the current image acquired by a probe is determined, the apparatus 100 may determine a diagnosis reliability level by analyzing and diagnosing the current image. The diagnosis reliability level may include not only a reliability level of a diagnostic result generated by analyzing and diagnosing the current image, but also a reliability level of the current diagnostic algorithm now performing diagnosis in the diagnostic device.

Then, the apparatus 600 provides a user with the determined reliability level of the diagnostic algorithm in 1140.

Although the same image is analyzed, diagnostic results may differ according to characteristics of a diagnostic algorithm being used. Thus, a diagnostic result obtained by using a diagnostic algorithm may differ according to a reliability level of a received image. According to the exemplary embodiment of the present disclosure, it is possible to provide a user with information whether a current diagnostic algorithm is suitable according to a reliability level of the currently received image. As a result the user may be able to change the diagnostic algorithm according to the image reliability level.

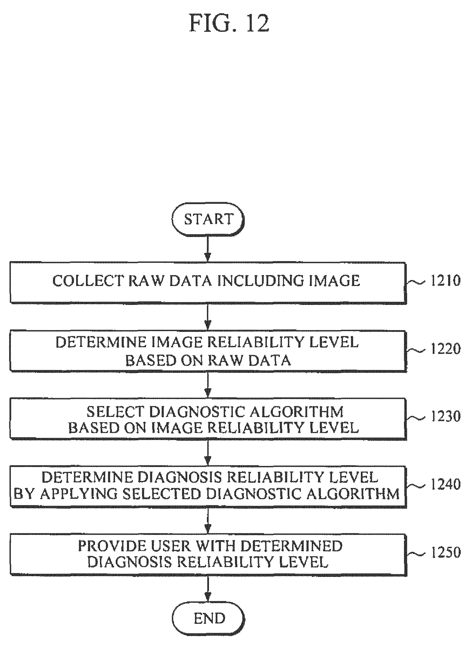

FIG. 12 is a flowchart illustrating a method for providing reliability according to another exemplary embodiment. FIG. 12 may be an example of a method implemented by the apparatus 700 shown in the example of FIG. 7 for providing reliability.

Referring to FIG. 12, the apparatus 700 collects raw data including an image acquired by a probe in 1210.

When the raw data is collected, the apparatus 700 generates an image reliability level using the collected raw data in 1220. At this point, as described above, the apparatus 700 may generate analysis information based on the raw data, and determine an image reliability level based on the generated analysis information. The image reliability level may be determined by applying the analysis information to a calculation algorithm or by referring to a pre-generated reliability database.

Based on the determined image reliability level which is determined for the currently received image, the apparatus 700 selects a diagnostic algorithm suitable for the currently received image in 1230.

Generally, various diagnostic algorithms may be used for Computer Aided Diagnosis (CAD), and they differ from each other in terms of diagnosis time and accuracy in a diagnostic result according to characteristics thereof. In the case of analyzing the currently received image with a low reliability level, if a diagnostic algorithm with high diagnosis speed but relatively low accuracy in a diagnostic result is used, a reliability level of the diagnostic result may be much lower. Therefore, the apparatus 700 may improve accuracy in a diagnostic result or a reliability level of the diagnostic result, by selecting a suitable diagnostic algorithm according to reliability level of the currently received image and analyzing the currently received image using the diagnostic algorithm.

Then, the apparatus 700 determines a diagnosis reliability level by applying the selected diagnostic algorithm in 1240. In this case, although a suitable diagnostic algorithm is selected according to a reliability level of the current image, a diagnostic result may not be obtained with the same level of the image reliability level because of characteristics of the diagnostic algorithm. Therefore, a reliability level of a diagnostic result, which is generated by applying the diagnostic algorithm, may be determined by considering the generated analysis information, the image reliability level, and a reliability level of the selected diagnostic algorithm.

In 1250, the apparatus 700 may output the image reliability level, the reliability level of the selected diagnostic algorithm, and the reliability level of a diagnostic result obtained by using the selected diagnostic algorithm to output devices, such as a probe, a diagnostic device, a diagnostic display device, and the like.

The methods and operations described above may be recorded, stored, or fixed in one or more computer-readable storage media that includes program instructions to be implemented by a computer to cause a processor to execute or perform the program instructions. The media may also include, alone or in combination with the program instructions, data files, data structures, and the like. Examples of computer-readable storage media include magnetic media, such as hard disks, floppy disks, and magnetic tape; optical media such as CD ROM disks and DVDs; magneto-optical media, such as optical disks; and hardware devices that are specially configured to store and perform program instructions, such as read-only memory (ROM), random access memory (RAM), flash memory, and the like. Examples of program instructions include machine code, such as produced by a compiler, and files containing higher level code that may be executed by the computer using an interpreter. The described hardware devices may be configured to act as one or more software modules in order to perform the operations and methods described above, or vice versa. In addition, a computer-readable storage medium may be distributed among computer systems connected through a network and computer-readable codes or program instructions may be stored and executed in a decentralized manner.

A number of examples have been described above. Nevertheless, it should be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. An apparatus for providing reliability information for Computer Aided Diagnosis (CAD), comprising:

a raw data collector configured to collect raw data, the raw data including an image acquired by a probe;
an image reliability determiner configured to determine a reliability level of the image using the collected raw data; and
a reliability provider configured to provide a user with reliability information corresponding to the determined reliability level of the image.

2. The apparatus of claim 1, wherein the raw data collector is further configured to collect the raw data from at least one from among a sensor installed in the probe or a diagnostic device, a wearable device worn by the user, and a device installed within a diagnostic screening room.

3. The apparatus of claim 1, wherein the image reliability determiner is further configured to generate analysis information based on the collected raw data, and to determine the reliability level of the image based on the generated analysis information.

4. The apparatus of claim 3, wherein the analysis information comprises at least one from among a moving speed of the probe, a degree of blurriness of the image, a measurement depth of the probe, and an angular velocity of the probe.

5. The apparatus of claim 3, wherein the image reliability determiner is further configured to generate the analysis information based on a difference between the image and a previous image.

6. The apparatus of claim 3, wherein the image reliability determiner is further configured to calculate a reliability level of the image by applying the generated analysis information to a calculation algorithm.

7. The apparatus of claim 3, wherein the image reliability determiner is further configured to determine the reliability level of the image by searching a reliability database using the analysis information.

8. The apparatus of claim 1, wherein the reliability information includes at least one from among visual information or non-visual information, and the reliability provider is further configured to output the reliability information to an output device,
wherein the visual information comprises at least one from among color information corresponding to the determined reliability level of the image, graph information corresponding to the determined reliability level of the image, and numeric information having a value indicative of the determined reliability level of the image,
wherein the non-visual information comprises at least one from among a predefined acoustic signal corresponding to the determined reliability level of the image, a predefined vibration signal corresponding to the determined reliability level of the image, and a voice signal regarding a numerical value indicative of the determined reliability of the image.

9. A method for providing reliability information for Computer Aided Diagnosis (CAD), comprising:

collecting raw data, the raw data including an image acquired by a probe;
determining a reliability level of the image based on the collected raw data; and
providing a user with reliability information corresponding to the determined reliability level of the image.

10. The method of claim 9, wherein the determining of a reliability level of the image comprises generating analysis information based on the collected raw data and determining the reliability level of the image based the generated analysis information.

11. The method of claim 10, wherein the analysis information comprises at least one from among a moving speed of the probe, a degree of blurriness of the image, measurement depth of the probe, and an angular velocity of the probe.

12. The method of claim 9, wherein the reliability information further comprises at least one from among visual information or non-visual information; and
the providing of the reliability information further comprises outputting the reliability information to an output device.

13. A computer-readable medium having instructions for providing diagnosis reliability information for Computer Aided Diagnosis (CAD), when performed by a processor, cause the processor to perform the steps of:

collecting raw data, the raw data including an image acquired by a probe;
determining a diagnosis reliability level of a diagnosis performed by a diagnostic device based on the collected raw data; and
providing a user with diagnosis reliability information corresponding to the determined diagnosis reliability level.

14. The computer-readable medium of claim 13, wherein the processor configured to perform the steps of:

generating analysis information based on the collected raw data; and
determining an image reliability level of the image based on the generated analysis information.

15. The computer-readable medium of claim 14, wherein the processor configured to perform the steps of:

selecting a diagnostic algorithm to be applied to the image based on the determined reliability level of the image.

FIG. 1

100

```
┌──────────────────────────────────────────────────────────────┐
│  ┌───────────────┐    ┌───────────────┐    ┌───────────────┐  │
│  │   RAW DATA    │───▶│     IMAGE     │───▶│  RELIABILITY  │  │
│  │   COLLECTOR   │    │  RELIABILITY  │    │   PROVIDER    │  │
│  │               │    │  DETERMINER   │    │               │  │
│  └───────────────┘    └───────────────┘    └───────────────┘  │
│          ⌐110                 ⌐120                 ⌐130         │
└──────────────────────────────────────────────────────────────┘
```

# FIG. 2A

210

```
+------------------------------------------------+
|  +----------------+        +----------------+   |
|  |   ANALYSIS     |        |     IMAGE      |   |
|  |  INFORMATION   | -----> |  RELIABILITY   |   |
|  |   GENERATOR    |        |   CALCULATOR   |   |
|  +----------------+        +----------------+   |
|              211                      212       |
+------------------------------------------------+
```

# FIG. 2B

220

ANALYSIS INFORMATION GENERATOR
221

IMAGE RELIABILITY EXTRACTOR
222

RELIABILITY DATABASE
223

FIG. 3A

FIG. 3B

FIG. 3C

330  331

RED  YELLOW  GREEN

FIG. 3D

RED | ORANGE | YELLOW
85%

330    331    332

FIG. 4

400

```
+-------------------------------------------------------------------+
|  +----------------+    +----------------+    +----------------+   |
|  |   RAW DATA     | -> |   DIAGNOSIS    | -> |  RELIABILITY   |   |
|  |   COLLECTOR    |    |  RELIABILITY   |    |   PROVIDER     |   |
|  |                |    |  DETERMINER    |    |                |   |
|  +----------------+    +----------------+    +----------------+   |
|          410                  420                   430          |
+-------------------------------------------------------------------+
```

FIG. 5A

510

ANALYSIS
INFORMATION
GENERATOR
511

DIAGNOSIS
RELIABILITY
CALCULATOR
512

FIG. 5B

FIG. 6

600

```
┌─────────────────────────────────────────────────────────┐
│  ┌──────────────────┐                                    │
│  │   RAW DATA       │                                    │
│  │   COLLECTOR      │                                    │
│  └──────────────────┘                                    │
│           └610                                           │
│           │                                              │
│           ▼                                              │
│  ┌──────────────┐    ┌──────────────┐    ┌─────────────┐ │
│  │    IMAGE     │    │  DIAGNOSIS   │    │ RELIABILITY │ │
│  │ RELIABILITY  │───▶│ RELIABILITY  │───▶│  PROVIDER   │ │
│  │ DETERMINER   │    │ DETERMINER   │    │             │ │
│  └──────────────┘    └──────────────┘    └─────────────┘ │
│        └620               └630                └640        │
└─────────────────────────────────────────────────────────┘
```

FIG. 7

700

```
┌ ─ ─ ─ ─ ─ ─ ─ ─ ─ ─ ─ ─ ─ ─ ─ ─ ─ ─ ─ ─ ─ ─ ─ ─ ─ ┐
│  ┌─────────────┐                                   │
│  │  RAW DATA   │                                   │
│  │  COLLECTOR  │                                   │
│  └─────────────┘                                   │
│         └710                                       │
│  ┌─────────────┐      ┌─────────────┐              │
│  │   IMAGE     │      │  DIAGNOSTIC │              │
│  │ RELIABILITY │─────▶│  ALGORITHM  │              │
│  │ DETERMINER  │      │   SELECTOR  │              │
│  └─────────────┘      └─────────────┘              │
│         └720                 └730                  │
│               ┌─────────────┐      ┌─────────────┐ │
│               │  DIAGNOSIS  │      │ RELIABILITY │ │
│               │ RELIABILITY │─────▶│  PROVIDER   │ │
│               │ DETERMINER  │      │             │ │
│               └─────────────┘      └─────────────┘ │
│                      └740                 └750     │
└ ─ ─ ─ ─ ─ ─ ─ ─ ─ ─ ─ ─ ─ ─ ─ ─ ─ ─ ─ ─ ─ ─ ─ ─ ─ ┘
```

# FIG. 8

```
         ┌─────────┐
         │  START  │
         └─────────┘
              │
              ▼
┌─────────────────────────────────────┐
│  COLLECT RAW DATA INCLUDING IMAGE    │──~ 810
└─────────────────────────────────────┘
              │
              ▼
┌─────────────────────────────────────┐
│  DETERMINE IMAGE RELIABILITY LEVEL   │──~ 820
│       BASED ON RAW DATA              │
└─────────────────────────────────────┘
              │
              ▼
┌─────────────────────────────────────┐
│           PROVIDE USER               │
│ WITH DETERMINED IMAGE RELIABILITY LEVEL │──~ 830
└─────────────────────────────────────┘
              │
              ▼
         ┌─────────┐
         │   END   │
         └─────────┘
```

# FIG. 9

```
START
  ↓
COLLECT RAW DATA
INCLUDING IMAGE               ~911
  ↓
DOES ANALYSIS          YES
INFORMATION EXIST?  ─────────┐
       ~912                   │
  │ NO                        │
  ↓                           │
GENERATE ANALYSIS            │
INFORMATION BASED ON RAW DATA │
       ~913                   │
  ↓←──────────────────────────┘
  NO    DOES RELIABILITY    YES
 ┌───── DATABASE EXIST? ──────┐
 │           ~914             │
 ↓                            ↓
CALCULATE IMAGE RELIABILITY   EXTRACT IMAGE RELIABILITY
LEVEL BY APPLYING ANALYSIS    LEVEL CORRESPONDING
INFORMATION TO CALCULATION    TO ANALYSIS INFORMATION
ALGORITHM                     FROM RELIABILITY DATABASE
       ~915                          ~916
 └────────────┬─────────────────────┘
              ↓
PROVIDE USER WITH DETERMINED
IMAGE RELIABILITY LEVEL
              ~917
              ↓
            END
```

FIG. 10

```
        ┌─────────┐
        │  START  │
        └────┬────┘
             │
┌────────────▼─────────────────────┐
│ COLLECT RAW DATA INCLUDING IMAGE │ ～ 1010
└────────────┬─────────────────────┘
             │
┌────────────▼─────────────────────┐
│ DETERMINE DIAGNOSIS RELIABILITY  │
│ LEVEL BASED ON RAW DATA          │ ～ 1020
└────────────┬─────────────────────┘
             │
┌────────────▼─────────────────────┐
│ PROVIDE USER WITH DETERMINED     │
│ DIAGNOSIS RELIABILITY LEVEL      │ ～ 1030
└────────────┬─────────────────────┘
             │
        ┌────▼────┐
        │   END   │
        └─────────┘
```

FIG. 11

START

COLLECT RAW DATA INCLUDING IMAGE ~1110

DETERMINE IMAGE RELIABILITY LEVEL
BASED ON RAW DATA ~1120

DETERMINE DIAGNOSIS RELIABILITY LEVEL
BASED ON IMAGE RELIABILITY LEVEL ~1130

PROVIDE USER WITH DETERMINED
DIAGNOSIS RELIABILITY LEVEL ~1140

END

FIG. 12

START

COLLECT RAW DATA INCLUDING IMAGE ~ 1210

DETERMINE IMAGE RELIABILITY LEVEL
BASED ON RAW DATA ~ 1220

SELECT DIAGNOSTIC ALGORITHM
BASED ON IMAGE RELIABILITY LEVEL ~ 1230

DETERMINE DIAGNOSIS RELIABILITY LEVEL
BY APPLYING SELECTED DIAGNOSTIC ALGORITHM ~ 1240

PROVIDE USER WITH DETERMINED
DIAGNOSIS RELIABILITY LEVEL ~ 1250

END

Europäisches
Patentamt

European
Patent Office

Office européen
des brevets

# EUROPEAN SEARCH REPORT

**Application Number**

EP 16 15 6616

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/246521 A1 (LUO HUI [US] ET AL) 6 October 2011 (2011-10-06) * paragraphs [0044] - [0102]; claim 1 * * claim 1 * * figure 1 * ----- | 1-15 | INV. G06F19/00 |

TECHNICAL FIELDS
SEARCHED      (IPC)

G06F

The present search report has been drawn up for all claims

| Place of search | Date of completion of the search | Examiner |
|---|---|---|
| Munich | 26 July 2016 | Rinelli, Pietro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 15 6616

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2016

| Patent document cited in search report | Publication date | Patent family member(s) | Publication date |
|---|---|---|---|
| US 2011246521 A1 | 06-10-2011 | NONE | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020150030384